# EUROPEAN PATENT SPECIFICATION

(11) **EP 4 312 821 B1**
(45) Date of publication and mention of the grant of the patent: **17.09.2025**
(21) Application number: 21716373.2
(22) Date of filing: 31.03.2021
(51) Int. Cl.: A61B 17/16, A61B 18/08

(54) **SET OF SURGICAL TOOLS FOR SPINAL FACET THERAPY**
SATZ VON CHIRURGISCHEN INSTRUMENTEN FÜR DIE WIRBELSÄULENFACETTENTHERAPIE
ENSEMBLE D'OUTILS CHIRURGICAUX POUR THÉRAPIE DE FACETTE VERTÉBRALE

(43) Date of publication of application: 07.02.2024
(73) Proprietor: Hoogland Spine Products GmbH, 85774 Unterföhring (DE)
(72) Inventor: HOOGLAND, Jaap, 85774 Unterföhring (DE); KEMMSTEDT, Dirk, 81827 München (DE)
(74) Representative: Hoffmann Eitle
(86) International application number: PCT/EP2021/058516
(87) International publication number: WO 2022/207105

(56) References cited:
- US-A1- 2008 091 207
- US-A1- 2010 331 883
- US-A1- 2014 012 261
- US-A1- 2018 103 964

## Description

### TECHNICAL FIELD

The present invention relates to a set of surgical tools for spinal facet therapy. In particular, the present invention relates to a set of surgical tools for use, for example, in the field of minimally invasive spinal treatment and/or surgery for facet joint syndrome using mechanical ablation and electromagnetic ablation and/or coagulation. Further, the present disclosure relates to a method (not claimed) for treating back pain, in particular treating facet joint syndrome, using mechanical ablation and electromagnetic ablation and/or coagulation.

### TECHNICAL BACKGROUND

To understand what is facet joint syndrome, one must first understand where it is located. The facet joint is located between two vertebrae in the spine. It is the joint that allows the back to be flexible and lets it bend and twist. The facet joint is unique in that it has innervations via a single nerve source. Nerves of the spinal cord exit through facet joints. In a healthy joint, the cartilage and synovial fluid that lubricates the joints allow the spine to move smoothly without too much grinding.

Facet joint syndrome can occur due to several factors including increased age, overload on joints (can be excess weight and occupation), or injury. These factors cause the discs between the vertebrae to degenerate and may eventually collapse leading to the narrowing of space. The excessive pressure on the surface of the facet joint leads to more damage and bone is left to grind on bone. Patients will have pain when there is a movement that involves the spine.

Conventionally, a process of facet joint rhizotomy or radiofrequency lesioning (RFL) has been utilized to provide temporary relief of spinal arthritis pain. RFL procedures involve cryotherapy or radiofrequency techniques to either freeze or burn the nerve. RFL is temporary because the nerve is destroyed at a point between the dorsal root ganglion (the nerve cell's body) and the end plate receptors (pain stimulation points on the joint) and thus, like any peripheral nerve, the nerve gradually regenerates, and the pain eventually returns. Hence, most RFL procedures last between 4 and 8 months and must be repeated when the pain returns for the rest of the patient's life for effective pain relief.

With regard to facet joint syndrome, surgery is usually only considered as an option when conservative treatment options can no longer help the patient. Traditional open surgery for facet joint syndrome can be complicated as it requires a large incision with a higher risk of postsurgical complications. It also involves a longer recovery and rehabilitation process.

When conducting a spinal surgery, it is a need to expose the intraspinal structures by removing parts of the facet joints and the facet joint capsule by a posterior or posterolateral approach. This removal of parts of the bone structure of the spine is carried out in order to access the spinal canal which is filled with nerve structures. The nerve structures in the spinal canal have a weak tissue structure and a tree-like configuration such that the central part of the spinal canal is filled with a long sack (dura) filled with nerve fibres. From this long sack filled with nerves, nerve roots are branching off at intervertebral levels to the left and to the right, whereas the nerve roots are leaving the spinal canal through bilateral tunnels, the foramen.

In the human being, the dural sack comprising the nerve fibres and its branches can be pathologically compressed at the level of the spinal canal or inside the foramen (tunnels) by:
- protruded or extruded disc material
- hypertrophied faced joints
- hypertrophied faced joint capsule
- synovial cysts from the facet joints
- intraspinal tumors or
- disc collapses that may cause narrowing of the foramen.

Such compression of the dural sack and the respective nerve fibres inside the dural sack causes pain, numbness or even paralysis of the patient. The most common complication is that of a herniated vertebral disc.

In order to reduce the risk of postsurgical complications, in recent years minimally invasive spinal surgery for facet joint syndrome has become more preferred, since it lowers complication rates and can be performed as an outpatient procedure, allowing patients to return to the comfort of their own homes on the day after the surgery itself. Procedures for facet joint syndrome includes any minimally invasive decompression surgery such as: facet thermal ablation, discectomy, laminotomy and foraminotomy.

Discectomy aims to remove the part of the disc that is causing compression. Hence, said facet treatment can be described as a debridement procedure that can be performed on a cervical, thoracic or lumbar facet joint of a human spine. During facet debridement, the synovial capsule between facets is removed so as to denude the bone and denervate the joint (preventing reinnervation).

To access the spinal canal in order to treat or remove the pathological tissue, the surrounding bone material has to be removed. By removing parts of the facet joint capsule and parts of the facet joint bone an opening of 6 to 11 mm diameter can be created through which a working cannula can be inserted. Through this working cannula the surgeon can manipulate the pathological interspinal tissue and can, for example, remove such tissue with standard instruments like graspers, curettes, reamers, lasers etc. Through such working cannula, also a cleaning of the interdiscal space can be performed and, if necessary, interdiscal spacers or cages can be inserted. After a working cannula is introduced, the removal of pathological structures in the spinal canal can, furthermore, be performed with aid of an endoscope.

Such surgery is usually carried out under the control of a two-direction X-ray image intensifier, also called "fluoroscopy".

The removal of the bone structure to access the spinal canal is a very delicate and sensitive procedure. It has to be avoided by any means that the nerve sack (dura) or one of the branching nerve roots is accidently injured or severed since such injury is very dangerous and the patient may not recover.

For carrying out such minimally invasive spinal treatment and/or surgery US 2019/0343575 A1 describes a spinal facet therapy system. The system includes an electrosurgical generator having a defined operational power curve with a maximum wattage of 60 Watts and a spinal facet therapy tool with an elongate rotatable shaft. The shaft has a distal end
with a cautery element. The tool is in communication with the electrosurgical generator and is configured to automatically rotate at between about 10 rpm to about 5000 rpm to remove an end plate receptor region with the synovial capsule of the spinal facet joint. The electrosurgical generator or RF power generator supplies power to the cautery element while the shaft rotates or is stationary.

The described system has the advantage that with one tool or system a denudement, debridement, coagulation and/or cauterization can be performed. However, many surgeons prefer performing denudement and/or debridement of the spinal facet joint or capsular via a surgical tool such as a drill or reamer providing a haptic feedback. Moreover, surgeons prefer removing nerves while using an endoscope for visualization of the treatment or surgery area.

Other related art may be found in US 2010/331883 A1 describing methods and systems for precisely placing and/or manipulating devices within the body by first positioning a guidewire or pullwire through the body from a first location, around a curved pathway, and out of the body through a second location, so that the distal and proximal ends of the guidewire extend from the body, then pulling a device into position using the guidewire, wherein the device to be positioned within the body is coupled to the proximal end of the guidewire, and the device is pulled into the body by pulling on the distal end of the guidewire that extends from the body. Further related art may be found in US 2018/103964 A1 describing system and methods for channeling a path into bone include a trocar having a proximal end, distal end and a central channel disposed along a central axis of the trocar.

### SUMMARY OF THE INVENTION

In view of the above, it would be desirable to provide a set of surgical tools that allows a minimally invasive spinal treatment and/or surgery for facet joint syndrome in which it is possible to visualize the treatment or surgery area during certain steps of the procedure and to allow a surgeon to carry out denudement and/or debridement via a surgical tool providing haptic feedback.

These desires are addressed by a set of surgical tools for spinal facet therapy for alleviating spinal pain according to claim 1. Embodiments may be found in the dependent claims, the following description and the accompanying drawings.

Associated methods are also described herein to aid understanding of the invention, but these do not form part of the claimed invention

The present invention provides a set of surgical tools for spinal facet therapy for alleviating spinal pain, in particular for minimally invasive spinal facet joint or capsular treatment and/or surgery using mechanical ablation and electromagnetic ablation and/or coagulation, comprising:
a first tool for performing a mechanic ablation of a part or parts of a target spinal facet joint or capsular, and
a second tool using electromagnetic waves for (thermal) ablation of tissue and/or nerves and/or parts of the target spinal facet joint or capsular and/or coagulation of tissue and/or parts of the target spinal facet join or capsular,
wherein the first tool is configured to be driven by a hand-held grip.

Hence, the inventio provides a set of surgical tools for carrying out a spinal facet therapy, in particular a minimally invasive spinal facet joint or capsular treatment and/or surgery, which set is capable of providing a surgeon with a haptic feedback while performing the treatment or surgery, in particular while conducting a denudement and/or debridement. Thereby, making it possible to remove parts of the synovial capsule and outer surface of the spinal facet joint in a gentle manner while protecting nerves, tissue and/or dura during the medical procedure, facilitating medical treatment besides extremely sensitive areas of an organism or human body so as to accelerate the healing process after surgery.

In the context of the present invention, the term "denudement" with regard to the conducted treatment or surgery is to be understood such that it refers to a procedure to polish, (gently) grind, scrape, file, grate, cleanse and/or rasp away soft tissue of facet joints to thereby denude tissue and uncover or expose the underlying bone without cutting into or removing the bone (e.g., in contrast to a sharp cutting edge like a knife). The denudement tool can have a surface that has an abrasive texture and/or configuration which may include small teeth. On the other hand, the term "debridement" refers in the context of the present invention to the removal of soft tissue associated with an end plate receptor region of a target spinal facet joint including the synovial capsule and tissue scraping of an outer bony surface of the joint.

Moreover, in the context of the present invention, the term "electromagnetic ablation" refers to thermal ablation that is achieved by application of an electromagnetic radiation (wave) such as radio waves, microwaves, infrared, (visible (light), ultraviolet, X-rays, and gamma rays. In this context, the use of radio waves, in particular bipolar radio waves, has proven to be extremely practicable and efficient.

Generally, embodiments of the present invention allow spinal facet joint debridement to remove the end plate receptor region which includes the synovial capsule and outer surface of the spinal facet joint. In case the synovial capsule and outer surface of the joint are denuded, the nerves have nowhere to re-adhere to the joint and thus the joint is permanently denervated. In other words, the patient is permanently relieved from pain.

The of surgical tools further includes:
a guide wire configured to be inserted into an incision provided on the back of a patient until it reaches the target spinal facet joint or capsular, and/or
a dilator configured to be put on or over the guide wire and guided by the guide wire towards the target spinal facet joint or capsular, and
a sleeve configured to be inserted into the incision over the guide wire and/or dilator until it reaches the target spinal facet joint or capsular and to provide a working channel.

Furthermore, in some embodiments, the set of surgical tools may further include an endoscope configured to be insertable into the working channel of the sleeve and used for visualizing the target spinal facet joint or capsular before and/or after performing mechanic and/or electromagnetic ablation.

Hence, the endoscope is provided or advanced through the working channel of the sleeve towards the target spinal facet joint or capsular and the second tool, in particular the RF probe, is provided or advanced through the endoscope inserted in the working channel so that the electromagnetic ablation takes place under vision control by the endoscope.

The sleeve includes a shaft extending longitudinally along the length of the sleeve and a fixation section for fixing or securing the sleeve on the target spinal facet joint or capsular, the fixation section being situated at a distal portion of the shaft, wherein the fixation section is preferably provided at or in a lateral surface of the shaft so as to extend along part of the circumference of the shaft.

Hence, making it possible to fixate the sleeve and thereby secure the treatment and/or surgery area on the target spinal facet joint or capsular, in particular once the correct position has been confirmed via fluoroscopy.

Moreover, in the context of the present invention, the terms "distal" and "proximal" are used in the sense that is usually assigned to them in connection with surgical instruments, namely the term "distal" indicating the end of a surgical instrument remote from the surgeon when the instrument is in use and the term "proximal" indicating the end of the surgical instrument that is near to a surgeon when the surgical instrument is in use. In other words, the distal end is the end of the surgical instrument which is inserted first into a patient's body and which typically is the end for manipulating the respective surgery side and the proximal end being in the hands of the surgeon.

Furthermore, the fixation section may include at least one cut that extends from the distal end of the sleeve for a predetermined distance along the longitudinal direction of the sleeve towards the proximal end of the sleeve.

Additionally, in some embodiments of the present invention, the at least one cut, preferably 3 cuts, may have a length of 1 to 4 mm, preferably 2 to 3 mm, and a width of 1 to 3 mm, preferably 1.5 to 2.5 mm. The length of the cut being parallel to the longitudinal direction of the sleeve and the width being perpendicular to the longitudinal direction of the sleeve.

Furthermore, in some embodiments of the present invention, the fixation section includes at least one fixing pin provided on the lateral surface of the shaft so as to extend along the longitudinal direction of the sleeve. Here, the at least one fixing pin preferably protrudes from the end face of the sleeve at the distal end, wherein the fixing pin preferably protrudes in a range of 0.2 mm to 2 mm, more preferably in a range of 0.5 mm to 1.5 mm.

In some embodiments, the first tool for performing mechanic ablation may include a drill member having a cutting section for cutting bones, said cutting section being situated at a distal end of the drill member.

Moreover, in some embodiments of the present invention, the drill member may include a non-cutting protection tip for protecting nerves, tissue or dura from being cut, the non-cutting protection tip being situated distally of the cutting section.

Furthermore, in some embodiments of the present invention, the cutting section comprises a plurality of cutting edges provided on the front surface of the drill member (110A) and extending radially outward.

In some embodiments, the first tool for performing mechanical ablation may include a handle situated at a proximal end of the first tool, in particular at a proximal end of a drill member of the first tool. Moreover, the handle may have a spherical or curved shape to fit comfortably in a hand of a surgeon. Moreover, the handle might be removable or detachable fixed to the first tool, in particular by means of a screwing connection.

Furthermore, the handle of the first tool, in particular the drill member, may have a spherical or curved potion followed by a straight portion, thereby, forming a mushroom shape. Moreover, both portions may be hollow, wherein the spherical or curved portion may additionally include a plurality of through-holes. Thereby, it becomes possible to reduce the overall weight of the handle and make it easy to grasp. Additionally, the straight portion might be provided on the outer circumference thereof with notches, preferably extending parallel to a longitudinal direction of the first tool, in particular a shaft thereof.

Furthermore, in some embodiments of the present invention, the handle has at least partially a spherical or curved shape with a radius in the range of 25 mm to 40 mm, preferably in the range of 30 mm to 35 mm. By providing the handle with a spherical or curved shape, it is possible to improve and/or enhance the application of force in comparison with a conventional straight handle, in particular the surgeon receives an improved feedback with regard to the applied force.

Accordingly, in some embodiments, the handle might be configured to provide the surgeon or operator with a haptic feedback, in particular with regard to the introduced or applied force and/or torque.

According to a further embodiment of the present invention, the second tool may include an electromagnetic probe or RF (radiofrequency) probe or a cautery element. Moreover, the second tool may be provided with an electrosurgical generator supplying power to the electromagnetic probe, the RF probe or the cautery element.

Moreover, the inner diameter of the working channel of the sleeve may be such that when the first tool, in particular the drill member, is inserted or received, the first tool or its drill member can rotate.

A not claimed method for treating back pain is provided, in particular by carrying out a minimally invasive spinal treatment and/or surgery using mechanical ablation and electromagnetic ablation and/or coagulation, by using the set of surgical tools according to any one of the preceding claims.

The method includes the steps of:
- advancing a first tool for performing mechanic ablation of a targeted spinal facet joint or capsular, and/or
- advancing a second tool using electromagnetic waves, in particular radio (frequency) waves, more particular bipolar radio waves, for ablating tissue and/or nerves and/or parts of a targeted spinal facet joint or capsular and/or coagulating tissue and/or parts of a targeted spinal facet joint or capsular,
wherein the first tool and the second tool are used one after the other, where the order depends on the treatment and/or surgery carried out.

In other words, the first tool and the second tool are used separately. Meaning, either the first tool or the second tool is inserted into the working channel of the sleeve, which has been placed before. Moreover, the order of using the first tool and the second tool, or the other way around, depends on the respective treatment or surgery carried out. Accordingly, it is possible to use at first the second tool for ablating or removing nerves, then use the first tool for mechanically removing parts of the targeted spinal facet joint or capsular, in particular by driving the drill element manually by hand, remove the first tool from the sleeve and inspect the targeted spinal facet joint or capsular via an endoscope, and if necessary, coagulate the spinal facet joint or the capsular by using the second tool once more.

The method includes the following steps:
- in a first step, the second tool (120) is used for ablating tissue and/or nerves of the targeted spinal facet joint or capsular,
- in a second step, the first tool (110) is used for ablating or shaving the targeted spinal facet joint or capsular, and
- in a third step, the second tool (120) is used for coagulating tissue and/or parts of the targeted spinal facet joint or capsular.

As mentioned above, an endoscope may be used before and/or after the use of the first tool and/or the use of the second tool, for inspecting and/or visualizing and/or recording the targeted spinal facet joint or capsular or treatment and/or surgical area.

Moreover, the second tool might be provided or advanced through the endoscope, in particular through a working channel of the endoscope, preferably under vision provided by the endoscope.

The endoscope might be used for water irrigation, in particular of the targeted spinal facet joint or capsular, thereby providing the necessary fluid for the bipolar RF probe.

Furthermore, the first tool and/or the second tool and the endoscope may be used simultaneously or at the same time, in particular they may be inserted into a working channel of a sleeve together, for inspection or visualization of the targeted spinal facet joint or capsular or treatment and/or surgical area before and/or during ablation via the first tool and/or the second tool.

Additionally, the first tool, in particular the drill element, may advance or may be advanced into the pathological tissue such as hypertrophied capsule of the facet joint or bone, synovial cyst or tumour is removed.

Furthermore, at first a guide wire, in particular a K-wire, may be inserted into an incision, which preferably takes into account the angle of approach required to reach the targeted spinal facet joint or capsular, the guide wire may be advanced into the incision until it reaches the targeted spinal facet joint or capsular, and then the guide wire is gently taped into the targeted spinal facet joint or capsular.

Additionally, in some embodiments the K-wire might have a diameter in the range of 1.2 mm to 1.8 mm, preferably 1.5 mm. By increasing the diameter of the K-wire to be in the range of 1.2 mm to 1.8 mm it becomes possible to improve the guiding stability of the K-wire and to improve the anchoring of the K-wire.

Moreover, the not claimed method may further include a step in which after the guide wire is positioned and the position has preferably been confirmed via fluoroscopy, a dilator is advanced over the guide wire towards the targeted spinal facet joint or capsular and the correct position of the guide wire and the dilator is preferably confirmed via fluoroscopy.

The method may further include the step of inserting and advancing a sleeve including a working channel over the dilator and the guide wire towards the targeted spinal facet joint or capsular and preferably fixate the sleeve at the targeted spinal facet joint or capsular by means of a fixation section provided on the sleeve.

The method for treating back pain, in particular by carrying out a minimally invasive spinal treatment and/or surgery using mechanical ablation and electromagnetic ablation and/or coagulation, may use the set of surgical tools for spinal facet therapy for alleviating spinal pain of the present invention. Therefore, the further features disclosed in connection with the above description of the set of surgical tools may also be applied to the method for treating back pain and vice versa.

### BRIEF DESCRIPTION OF THE DRAWINGS

A more complete appreciation of the present invention and many of the attendant advantages thereof will be readily obtained as the same becomes better understood by reference to the following detailed description when considered in connection with the accompanying drawings, in which:
- Fig. 1: shows a schematic illustration of surgical tools during treatment of a targeted spinal facet joint, according to the prior art;
- Fig. 2: shows a schematic isometric sectional view of a part of the set of surgical tools according to a first embodiment of the present invention; and
- Fig. 3: shows schematic plane views of parts of a set of surgical tools according to a second embodiment of the present invention.

### DETAILED DESCRIPTION

Embodiments of the present disclosure will now be explained with reference to the drawings. It will be apparent to those skilled in the field of spinal medical procedures that the following description of the embodiments is provided for illustration only and not for the purpose of limiting the disclosure. Features of the embodiments described below can also be used to further characterize the devices defined in the claims.

Modifications of features can be combined to form further embodiments. Features described in individual embodiments can be provided in a single embodiment if they are not incompatible. Likewise, features described in a single embodiment can be provided in several embodiments individually or in any suitable sub-combination. As used in the specification and the appended claims, the singular forms "a", "an", "the" and the like include reference to a plurality, unless the context clearly dictates otherwise.

The same reference numerals listed in different figures refer to identical, corresponding or functionally similar elements. As described hereinafter, exemplary implementations of the present disclosure relate to a set of surgical tools for spinal facet therapy for alleviating spinal pain.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

Additionally, it will be understood that when an element is referred to as being "on", "attached" to, "connected" to, "coupled" with, "contacting", etc., another element, it can be directly on, attached to, connected to, coupled with or contacting the other element or intervening elements may also be present. In contrast, when an element is referred to as being, for example, "directly on," "directly attached" to, "directly connected" to, "directly coupled" with or "directly contacting" another element, there are no intervening elements present. It will also be appreciated by those of skill in the art that references to a structure or feature that is disposed "adjacent" another feature may have portions that overlap or underlie the adjacent feature.

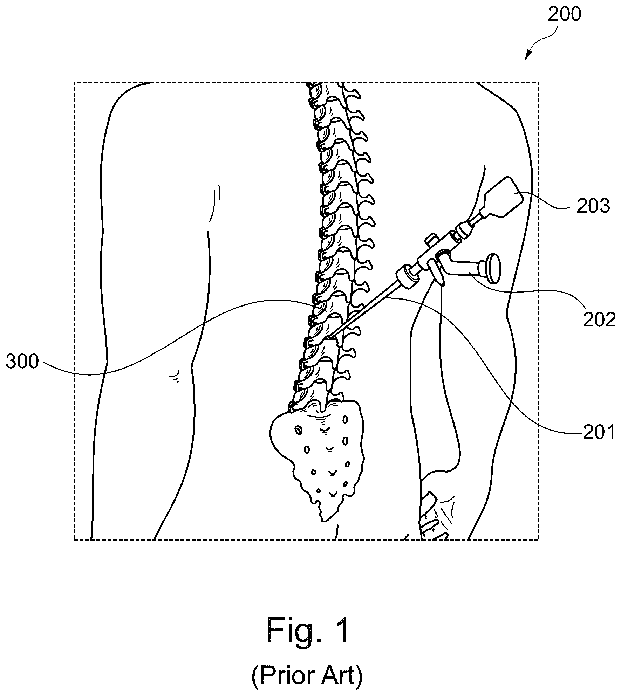

Figure 1 shows a schematic illustration of surgical tools 200 during treatment of a targeted spinal facet joint (spinal column 300), according to the prior art. As can be taken from Figure 1, conventional surgical tools 200 used in spinal facet therapy for alleviating spinal pain consist of a guide wire, a sleeve 201, an endoscope 202 and a surgical instrument like grasping forceps 203 or a drill. Conventionally, the patient is placed in a prone position, the treatment site(s) is draped and prepared following standard techniques, the desired treatment location is determined by using fluoroscopy and the skin is marked. In a next step, a 10 to 15 mm incision is made in a location that takes into account the angle of approach required to reach the targeted spinal facet joint. Then, using fluoroscopic guidance, a guide wire (K-wire) is advanced into the incision until it reaches the face joint. The guide wire is gently taped into the bone using a small hammer, confirming the correct position of the guide wire by using fluoroscopy. In a next step, a dilator is inserted over the guide wire to widen the provided channel for the sleeve, which is then inserted over the dilator or guide wire. Then, the guide wire and/or dilator are removed, so that a working channel within the sleeve is made accessible. If desired, an endoscope is inserted into the working channel for inspecting the targeted spinal facet joint or capsular. Figure 1 shows a surgical system in which the endoscope 202 can be used parallel to the grasping forceps 203. Making it possible for the surgeon to visualize the targeted spinal facet joint or capsular during treatment.

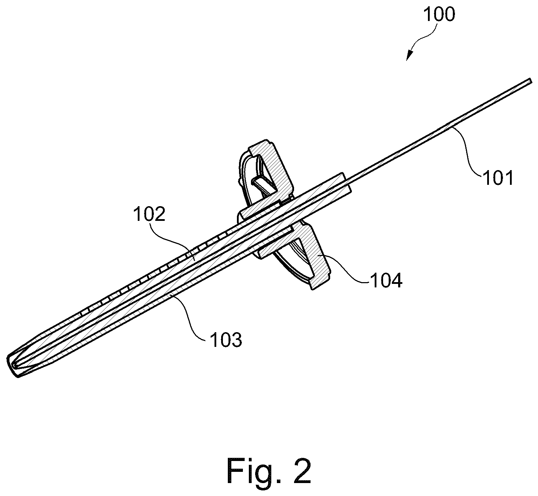

Figure 2 shows a schematic isometric sectional view of a part of the set of surgical tools 100 according to a first embodiment of the present invention. As can be taken from Figure 2, the set of surgical tools 100 includes a guide wire 101, in particular a K-wire, which is configured to be inserted into an incision provided on the back of a patient, as explained above with regard to Figure 1. The guide wire 101 can be advanced into the incision until it reaches the targeted spinal facet joint or capsular. After confirming the correct position of the guide-wire via fluoroscopy, its position is usually fixed by tapping the wire 101 gently into the bone using a small hammer. The set of surgical tools 100 also includes a dilator 102 that is configured to be put on or over the guide wire and guided by the guide wire during advancing it to the targeted spinal facet joint or capsular. Additionally, the set includes a sleeve '103 that is configured to be inserted into the incision over the guide wire 101 and/or the dilator 102, in case the guide wire 101 is removed before. As before, the sleeve 103 can be inserted into the incision or advanced until it reaches the targeted spinal facet joint or capsular. Moreover, the shown set of surgical tools 100 includes a hand grip 104 that is connected or connectable to the sleeve 103, making it easier for a surgeon to advance and/or position the sleeve 103 in the incision.

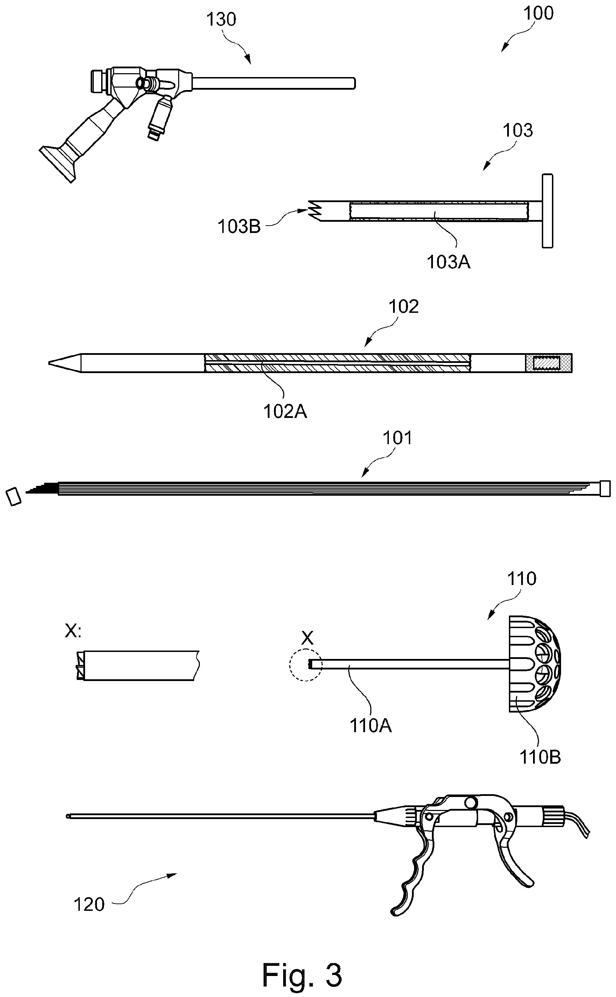

Figure 3 shows schematic plane views of parts of a set of surgical tools 100 according to a second embodiment of the present invention. As shown in Figure 3, the set further includes a first tool 110, in the shown embodiment a hand driven drill, and a second tool 120, in the shown embodiment an electromagnetic probe that can emit high electromagnetic waves or radio waves. The set further includes an endoscope 130, a number of guide wires or K-wires (101) and a dilator 102. It can also be taken from Figure 3 that the dilator 102 is provided with a channel, in particular a guide wire channel 102A that is used for guiding the dilator 102 via the guide wire 101 towards the targeted spinal facet joint or capsular.

The drill 110 is used for performing mechanical ablation for example of a part or parts of the targeted spinal facet joint or capsular. The second tool 120, in particular the electromagnetic probe, can be used for different purposes. Firstly, it can be used for coagulating the treated spinal facet joint or capsular. Additionally, if desired, it can also be used for ablation, in particular thermal ablation, of tissue and/or nerves and/or (small) parts of the targeted spinal facet joint or capsular. Since the provided set of surgical tools 100 provides the opportunity to use the endoscope 30 via the same sleeve 103, allowing to investigate the targeted treatment area, it becomes possible to perform at first a rough treatment via the mechanical ablation, examine the treatment area and if necessary or desired perform a fine thermal ablation via the second tool 120. Thereby, increasing the quality of the performed treatment and/or surgery and reducing the risk of possible injuries, in particular of nerves.

Moreover, as shown in Figure 3, the sleeve 103 includes a shaft extending longitudinally along the length of the sleeve 103. In other words, along the insertion direction of the sleeve 103 into the incision. The shaft is provided with a fixation section 103B that is configured for fixing or securing the sleeve 103 on the targeted spinal facet joint or capsular. As shown in Figure 3, the fixation section 103B is positioned at a distal portion of the shaft. The shown fixation section 103B consists of three cuts or recesses that extend from the distal end of the sleeve 103 for a predetermined distance in a range of 1 to 4 mm into the sleeve 103, in particular along the longitudinal direction of the sleeve 103. As also shown in Fig. 3, the sleeve 103 is further provided with a working channel 103A that is used for inserting or advancing the first tool 110, the second tool 120 and the endoscope 130. However, preferably the first tool 110 and the second tool 120 are inserted or advanced through the endoscope 130 which is also provided with a working channel (not shown).

As can also be taken from Figure 3, the drill 110 includes a drill member 110A having a cutting section able of cutting bones, wherein said cutting section is situated at a distal end of the drill member 110A. The shown drill 110 further includes a handle 110B provided on the proximal end of the drill member 110A, wherein the handle 110B has a spherical portion followed or changing (merging) into a straight portion, thereby giving the handle 110B a mushroom shape that comfortable fits into the hand of a surgeon. Both portions are made hollow, thereby reducing the weight of the handle 110B. Additionally, the spherical portion is provided with a plurality of holes, further reducing the weight of the handle 110B and improving the haptic of the handle 110B. Additionally, the straight portion includes on the outer circumference notches, which makes it easier for the surgeon to securely hold or grasp the handle 110B.

### LIST OF REFERENCE SIGNS

- 100: Set of surgical tools
- 101: Guide wire (K-wire)
- 102: Dilator
- 102A: Guide wire channel (Dilator)
- 103: Sleeve
- 103A: Working channel
- 103B: Fixation section
- 104: Hand grip
- 110: First tool (mechanical ablation tool)
- 110A: Drill member (first tool)
- 110B: Handle (first tool)
- 120: Second tool (electromagnetic ablation and/or coagulation tool
- 130: Endoscope

- 200: Surgical tools (Prior Art)
- 201: Sleeve
- 202: Endoscope
- 203: Grasping forceps

- 300: spinal column

## Claims

1. Set of surgical tools (100) for spinal facet therapy for alleviating spinal pain, in particular for minimally invasive spinal facet joint or capsular treatment and/or surgery using mechanical ablation and electromagnetic ablation and/or coagulation, comprising:
a first tool (110) configured to perform mechanic ablation of a part of a target spinal facet joint or capsular, and
a second tool (120) configured to use electromagnetic waves for ablation of tissue and/or nerves and/or parts of the target spinal facet joint or capsular and/or coagulation of tissue and/or parts of the target spinal facet joint or capsular,
wherein the first tool (110) is configured to be driven by a hand-held grip,the set of surgical tools (100) further comprising:a sleeve (103) configured to be inserted into the incision over a guide wire (101) and/or dilator (102) until it reaches the target spinal facet joint or capsular and to provide a working channel (103A),
wherein the sleeve (103) comprises a shaft extending longitudinally along the length of the sleeve (103) and a fixation section (103B) for fixing or securing the sleeve (103) on the target spinal facet joint or capsular, the fixation section (103B) being situated at a distal portion of the shaft.

2. The set of surgical tools (100) according to claim 1, further comprising:
a guide wire (101), in particular a K-wire, configured to be inserted into an incision provided on the back of a patient until it reaches the target spinal facet joint or capsular, and/or
a dilator (102) configured to be put on or over the guide wire and guided by the guide wire towards the target spinal facet joint or capsular.

3. The set of surgical tools (100) according to claim 1 or 2, further comprising:
an endoscope (130) configured to be insertable into the working channel (103A) and used for visualizing the target spinal facet joint or capsular before and/or after performing mechanic and/or electromagnetic (wave) ablation.

4. The set of surgical tools (100) according to any one of claims 1 to 3, wherein the fixation section (103B) is provided at or in a lateral surface of the shaft so as to extend along part of the circumference of the shaft.

5. The set of surgical tools (100) according to claim 4, wherein the fixation section includes at least one cut that extends from the distal end of the sleeve (103) for a predetermined distance along the longitudinal direction of the sleeve (103) towards the proximal end of the sleeve.

6. The set of surgical tools (100) according to claim 5, wherein the at least one cut, preferably 3 cuts, has or have a length of 1 to 4 mm, preferably 2 to 3 mm, and a width of 1 to 3 mm, preferably 1.5 to 2.5 mm.

7. The set of surgical tools (100) according to any one of claims 4 to 6, wherein the fixation section includes at least one fixing pin provided on the lateral surface of the shaft so as to extend along the longitudinal direction of the sleeve (103), wherein the at least one fixing pin protrudes from the end face of the sleeve (103) at the distal end, wherein the fixing pin preferably protrudes in a range of 0.2 mm to 2 mm, more preferably in a range of 0.5 mm to 1.5 mm.

8. The set of surgical tools according to any one of the preceding claims, wherein the first tool (110) for performing mechanic ablation includes a drill member (110A) having a cutting section for cutting bone, said cutting section being situated at a distal end of the drill member.

9. The set of surgical tools according to claim 7 or 8, wherein the cutting section comprises a plurality of cutting edges provided on the front surface of the drill member (110A) and extending radially outward.

10. The set of surgical tools according to any one of the preceding claims, wherein the first tool (110) for performing mechanical ablation includes a handle (110B) situated at a proximal end of the first tool (110), in particular at a proximal end of a drill member (110A) of the first tool (110), wherein the handle (110B) preferably has a spherical or curved shape to fit comfortably in a hand of a surgeon.

11. The set of surgical tools according to claim 10, wherein the handle (110B) has a spherical or curved portion followed by a straight portion, both portions being hollow, wherein the spherical or curved portion preferably includes through-holes and the straight portion preferably includes on the outer circumference notches.

12. The set of surgical tools according to claim 10 or 11, wherein the handle (110B) has at least partially a spherical or curved shape with a radius in the range of 25 mm to 40 mm, preferably, 30 mm to 35 mm.

13. The set of surgical tools according to any one of the preceding claims 10 to 12, wherein the handle is configured to provide the surgeon or operator with a haptic feedback, in particular with regard to the introduced force and/or torque.

14. The set of surgical tools according to any one of the preceding claims, wherein the second tool (120) includes an electromagnetic probe or RF (radiofrequency) probe or a cautery element, wherein the second tool (120) is preferably provided with an electrosurgical generator supplying power to the electromagnetic probe, the RF probe or the cautery element.

15. The set of surgical tools according to any one of the preceding claims, wherein the inner diameter of the working channel (103A) of the sleeve (103) is such that when the first tool (110), in particular the drill member (110A) is inserted or received, the first tool (110) or its drill member (110A) can rotate.

## Patentansprüche

1. Satz von chirurgischen Instrumenten (100) für die Wirbelsäulenfacettentherapie zur Linderung von Wirbelsäulenenschmerzen, insbesondere für eine minimalinvasive Behandlung und/oder Operation von Wirbelsäulenfacettentgelenken oder -kapseln unter Verwendung einer mechanischen Ablation und einer elektromagnetischen Ablation und/oder Koagulation, umfassend:
ein erstes Instrument (110), das dazu konfiguriert ist, eine mechanische Ablation eines Teils eines Zielwirbelsäulenfacettengelenks oder einer Zielwirbelsäulenfacettenkapsel durchzuführen, und
ein zweites Instrument (120), das dazu konfiguriert ist, elektromagnetische Wellen zur Ablation von Gewebe und/oder Nerven und/oder Teilen des Zielwirbelsäulenfacettengelenks oder der Zielwirbelsäulenfacettenkapsel und/oder zur Koagulation von Gewebe und/oder Teilen des Zielwirbelsäulenfacettengelenks oder der Zielwirbelsäulenfacettenkapsel zu verwenden,
wobei das erste Instrument (110) so konfiguriert ist, dass es durch einen Handgriff angetrieben wird, wobei der Satz von chirurgischen Instrumenten (100) weiter umfasst: eine Hülse (103), die so konfiguriert ist, dass sie in den Einschnitt über einen Führungsdraht (101) und/oder Dilatator (102) eingeführt wird, bis sie das Zielwirbelsäulenfacettengelenk oder die Zielwirbelsäulenfacettenkapsel erreicht, und einen Arbeitskanals (103A) bereitstellt,
wobei die Hülse (103) eine Welle, die sich in Längsrichtung entlang der Länge der Hülse (103) erstreckt, und einen Befestigungsabschnitt (103B) zum Befestigen oder Sichern der Hülse (103) am Zielwirbelsäulenfacettengelenk oder der Zielwirbelsäulenfacettenkapsel umfasst, wobei sich der Befestigungsabschnitt (103B) an einem distalen Abschnitt der Welle befindet.

2. Satz von chirurgischen Instrumenten (100) nach Anspruch 1, weiter umfassend:
einen Führungsdraht (101), insbesondere einen K-Draht, der so konfiguriert ist, dass er in einen Einschnitt, der auf dem Rücken eines Patienten vorgesehen ist, eingeführt wird, bis er das Zielwirbelsäulenfacettengelenk oder die Zielwirbelsäulenfacettenkapsel erreicht, und/oder
einen Dilatator (102), der so konfiguriert ist, dass er auf oder über den Führungsdraht gelegt wird und durch den Führungsdraht in Richtung des Zielwirbelsäulenfacettengelenks oder der Zielwirbelsäulenfacettenkapsel geführt wird.

3. Satz von chirurgischen Instrumenten (100) nach Anspruch 1 oder 2, weiter umfassend:
ein Endoskop (130), das so konfiguriert ist, dass es in den Arbeitskanal (103A) eingeführt werden kann und zur Visualisierung des Zielwirbelsäulenfacettengelenks oder der Zielwirbelsäulenfacettenkapsel vor und/oder nach einem Durchführen einer mechanischen und/oder elektromagnetischen (Wellen-)Ablation verwendet wird.

4. Satz von chirurgischen Instrumenten (100) nach einem der Ansprüche 1 bis 3, wobei der Befestigungsabschnitt (103B) an oder in einer laterlaen Oberfläche der Welle vorgesehen ist, so dass er sich entlang eines Teils des Umfangs der Welle erstreckt.

5. Satz von chirurgischen Instrumenten (100) nach Anspruch 4, wobei der Befestigungsabschnitt mindestens einen Schnitt einschließt, der sich vom distalen Ende der Hülse (103) über eine vorbestimmte Distanz entlang der Längsrichtung der Hülse (103) in Richtung des proximalen Endes der Hülse erstreckt.

6. Satz von chirurgischen Instrumenten (100) nach Anspruch 5, wobei der mindestens eine Schnitt, vorzugsweise 3 Schnitte, eine Länge von 1 bis 4 mm, vorzugsweise 2 bis 3 mm, und eine Breite von 1 bis 3 mm, vorzugsweise 1,5 bis 2,5 mm aufweist bzw. aufweisen.

7. Satz von chirurgischen Instrumenten (100) nach einem der Ansprüche 4 bis 6, wobei der Befestigungsabschnitt mindestens einen Befestigungsstift einschließt, der an der lateralen Oberfläche der Welle vorgesehen ist, so dass er sich entlang der Längsrichtung der Hülse (103) erstreckt, wobei der mindestens eine Befestigungsstift aus der Endfläche der Hülse (103) am distalen Ende herausragt, wobei der Befestigungsstift vorzugsweise in einem Bereich von 0,2 mm bis 2 mm, besonders bevorzugt in einem Bereich von 0,5 mm bis 1,5 mm hervorsteht.

8. Satz von chirurgischen Instrumenten nach einem der vorstehenden Ansprüche, wobei das erste Instrument (110) zum Durchführen einer mechanischen Ablation ein Bohrelement (110A) mit einem Schneidabschnitt zum Schneiden von Knochen einschließt, wobei sich der Schneidabschnitt an einem distalen Ende des Bohrelements befindet.

9. Satz von chirurgischen Instrumenten nach Anspruch 7 oder 8, wobei der Schneidabschnitt eine Vielzahl von Schneidkanten umfasst, die an der vorderen Oberfläche des Bohrelements (110A) vorgesehen sind und sich radial nach außen erstrecken.

10. Satz von chirurgischen Instrumenten nach einem der vorstehenden Ansprüche, wobei das erste Instrument (110) zum Durchführen einer mechanischen Ablation einen Griff (110B) einschließt, der sich an einem proximalen Ende des ersten Instruments (110) befindet, insbesondere an einem proximalen Ende eines Bohrelements (110A) des ersten Instruments (110), wobei der Griff (110B) vorzugsweise eine kugelförmige oder gekrümmte Form aufweist, damit er bequem in eine Hand eines Chirurgen passt.

11. Satz von chirurgischen Instrumenten nach Anspruch 10, wobei der Griff (110B) einen kugelförmigen oder gekrümmten Abschnitt aufweist, gefolgt von einem geraden Abschnitt, wobei beide Abschnitte hohl sind, wobei der kugelförmige oder gekrümmte Abschnitt vorzugsweise Durchgangslöcher einschließt und der gerade Abschnitt vorzugsweise am Außenumfang Kerben einschließt.

12. Satz von chirurgischen Instrumenten nach Anspruch 10 oder 11, wobei der Griff (110B) zumindest teilweise eine kugelförmige oder gekrümmte Form mit einem Radius im Bereich von 25 mm bis 40 mm, vorzugsweise 30 mm bis 35 mm, aufweist.

13. Satz von chirurgischen Instrumenten nach einem der vorstehenden Ansprüche 10 bis 12, wobei der Griff dazu konfiguriert ist, dem Chirurgen oder Bediener eine haptische Rückmeldung, insbesondere hinsichtlich der eingeleiteten Kraft und/oder des Drehmoments, zu liefern.

14. Satz von chirurgischen Instrumenten nach einem der vorstehenden Ansprüche, wobei das zweite Instrument (120) eine elektromagnetische Sonde oder eine HF-Sonde (Hochfrequenzsonde) oder ein Kauterelement einschließt, wobei das zweite Instrument (120) vorzugsweise mit einem elektrochirurgischen Generator ausgestattet ist, der die elektromagnetische Sonde, die HF-Sonde oder das Kauterelement mit Strom versorgt.

15. Satz von chirurgischen Instrumenten nach einem der vorstehenden Ansprüche, wobei der Innendurchmesser des Arbeitskanals (103A) der Hülse (103) derart ist, dass beim Einsetzen oder Aufnehmen des ersten Instruments (110), insbesondere des Bohrelements (110A), das erste Instrument (110) oder dessen Bohrelement (110A) rotieren kann.

## Revendications

1. Ensemble (100) d'outils chirurgicaux pour la thérapie de facettes vertébrales destinée à soulager les douleurs vertébrales, en particulier pour le traitement mini-invasif d'articulation ou de capsule facettaire vertébrale et/ou la chirurgie en utilisant une ablation mécanique et une ablation et/ou une coagulation électromagnétiques, comprenant :
un premier outil (110) configuré pour effectuer une ablation mécanique d'une partie d'une articulation ou d'une capsule facettaire vertébrale cible, et
un second outil (120) configuré pour utiliser des ondes électromagnétiques pour l'ablation de tissus et/ou de nerfs et/ou de parties de l'articulation ou de la capsule facettaire vertébrale cible et/ou la coagulation de tissus et/ou de parties de l'articulation ou de la capsule facettaire vertébrale cible,
dans lequel le premier outil (110) est configuré pour être entraîné par une poignée à main, l'ensemble (100) d'outils chirurgicaux comprenant en outre : un manchon (103) configuré pour être inséré dans l'incision sur un fil-guide (101) et/ou un dilatateur (102) jusqu'à ce qu'il atteigne l'articulation ou la capsule facettaire vertébrale cible et pour fournir un canal (103A) de travail,
dans lequel le manchon (103) comprend un axe s'étendant longitudinalement sur la longueur du manchon (103) et une section de fixation (103B) pour fixer ou immobiliser le manchon (103) sur l'articulation ou la capsule facettaire vertébrale cible, la section de fixation (103B) étant située au niveau d'une partie distale de l'axe.

2. Ensemble (100) d'outils chirurgicaux selon la revendication 1, comprenant en outre :
un fil-guide (101), en particulier une broche de Kirschner, configuré pour être inséré dans une incision pratiquée sur le dos d'un patient jusqu'à ce qu'il atteigne l'articulation ou la capsule facettaire vertébrale cible, et/ou
un dilatateur (102) configuré pour être placé sur ou au-dessus du fil-guide et guidé par le fil-guide vers l'articulation ou la capsule facettaire vertébrale cible.

3. Ensemble (100) d'outils chirurgicaux selon la revendication 1 ou la revendication 2, comprenant en outre :
un endoscope (130) configuré pour pouvoir être inséré dans le canal (103A) de travail et utilisé pour visualiser l'articulation ou la capsule facettaire vertébrale cible avant et/ou après la réalisation d'une ablation mécanique et/ou électromagnétique (par ondes).

4. Ensemble (100) d'outils chirurgicaux selon l'une quelconque des revendications 1 à 3, dans lequel la section de fixation (103B) est disposée au niveau ou dans une surface latérale de l'axe de manière à s'étendre le long d'une partie de la circonférence de l'axe.

5. Ensemble (100) d'outils chirurgicaux selon la revendication 4, dans lequel la section de fixation inclut au moins une découpe qui s'étend de l'extrémité distale du manchon (103) sur une distance prédéterminée le long de la direction longitudinale du manchon (103) vers l'extrémité proximale du manchon.

6. Ensemble (100) d'outils chirurgicaux selon la revendication 5, dans lequel l'au moins une découpe, de préférence 3 découpes, présente ou présentent une longueur de 1 à 4 mm, de préférence de 2 à 3 mm, et une largeur de 1 à 3 mm, de préférence de 1,5 à 2,5 mm.

7. Ensemble (100) d'outils chirurgicaux selon l'une quelconque des revendications 4 à 6, dans lequel la section de fixation inclut au moins une tige de fixation disposée sur la surface latérale de l'axe de manière à s'étendre le long de la direction longitudinale du manchon (103),
dans lequel l'au moins une tige de fixation fait saillie depuis la face d'extrémité du manchon (103) à l'extrémité distale, dans lequel la tige de fixation fait de préférence saillie dans une plage de 0,2 mm à 2 mm, plus préférentiellement dans une plage de 0,5 mm à 1,5 mm.

8. Ensemble d'outils chirurgicaux selon l'une quelconque des revendications précédentes, dans lequel le premier outil (110) pour réaliser une ablation mécanique inclut un élément foret (110A) présentant une section de coupe pour couper de l'os, ladite section de coupe étant située à une extrémité distale de l'élément foret.

9. Ensemble d'outils chirurgicaux selon la revendication 7 ou la revendication 8, dans lequel la section de coupe comprend une pluralité de bords de coupe disposés sur la surface avant de l'élément foret (110A) et s'étendant radialement vers l'extérieur.

10. Ensemble d'outils chirurgicaux selon l'une quelconque des revendications précédentes, dans lequel le premier outil (110) pour réaliser une ablation mécanique inclut une poignée (110B) située à une extrémité proximale du premier outil (110), en particulier à une extrémité proximale d'un élément foret (110A) du premier outil (110), dans lequel la poignée (110B) présente de préférence une forme sphérique ou incurvée pour tenir confortablement dans la main d'un chirurgien.

11. Ensemble d'outils chirurgicaux selon la revendication 10, dans lequel la poignée (110B) présente une partie sphérique ou incurvée suivie d'une partie droite, les deux parties étant creuses, dans lequel la partie sphérique ou incurvée inclut de préférence des trous traversants et la partie droite inclut de préférence des encoches sur la circonférence extérieure.

12. Ensemble d'outils chirurgicaux selon la revendication 10 ou la revendication 11, dans lequel la poignée (110B) est au moins partiellement de forme sphérique ou incurvée avec un rayon dans la plage de 25 mm à 40 mm, de préférence de 30 mm à 35 mm.

13. Ensemble d'outils chirurgicaux selon l'une quelconque des revendications 10 à 12 précédentes, dans lequel la poignée est configurée pour fournir au chirurgien ou à l'opérateur un retour haptique, en particulier concernant la force et/ou le couple introduits.

14. Ensemble d'outils chirurgicaux selon l'une quelconque des revendications précédentes, dans lequel le second outil (120) inclut une sonde électromagnétique ou une sonde RF (radiofréquence) ou un élément de cautérisation, dans lequel le second outil (120) est de préférence doté d'un générateur électrochirurgical fournissant de l'énergie à la sonde électromagnétique, à la sonde RF ou à l'élément de cautérisation.

15. Ensemble d'outils chirurgicaux selon l'une quelconque des revendications précédentes,
dans lequel le diamètre intérieur du canal (103A) de travail du manchon (103) est tel que lorsque le premier outil (110), en particulier l'élément foret (110A) est inséré ou reçu, le premier outil (110) ou son élément foret (110A) puisse tourner.
